# EUROPEAN PATENT SPECIFICATION

(11) **EP 0 604 454 B1**
(45) Date of publication and mention of the grant of the patent: **21.10.1998**
(21) Application number: 92916901.9
(22) Date of filing: 31.07.1992
(51) Int. Cl.: C08K 3/30, C08K 5/17, C08K 5/36, C08K 5/13, C08L 35/08, A61K 6/00

(54) **STABILIZED AQUEOUS SOLUTION**
STABILISIERTE WÄSSRIGE LÖSUNG
SOLUTION AQUEUSE STABILISEE

(30) Priority: 17.09.1991 US 761089; 18.09.1991 US 761535
(43) Date of publication of application: 06.07.1994
(60) Divisional application: 97114020.7
(73) Proprietor: ISP INVESTMENTS INC., Wilmington, Delaware 19801 (US)
(72) Inventor: PLOCHOCKA, Krystyna, Scotch Plains, NJ 07076 (US); CHUANG, Jui-Chang, Wayne, NJ 07470 (US); LOGIN, Robert, B., Oakland, NJ 07436 (US)
(74) Representative: Ford, Michael Frederick
(86) International application number: US9206298
(87) International publication number: WO9306165

(56) References cited:
- EP-A- 0 295 730
- US-A- 3 531 427
- US-A- 4 396 734
- US-A- 4 410 619
- US-A- 4 419 433
- US-A- 4 581 405
- US-A- 4 863 989

## Description

What is provided herein is a colorless, stabilized aqueous solution of a C₁-C₅ alkyl vinyl ether and maleic acid copolymer which includes 10-500 ppm of an inorganic reducing agent which is sulfur dioxide or sodium or potassium bisulfite, sodium or potassium metabisulfite, sodium or potassium thiosulfite, or sodium or potassium sulfite, said stabilized solution exhibiting a retention of at least about 85% of its original viscosity (Brookfield) after 2 months.

Preferably, the stabilized solution is prepared by hydrolyzing the corresponding anhydride in the presence of said additive.

In one embodiment, copolymers of maleic anhydride and a C₁-C₅ alkyl vinyl ether, e.g. methyl vinyl ether, are dissolved in water containing about 10-500 ppm of a stabilizer additive which is sulfur dioxide, sodium or potassium bisulfite, sodium or potassium metabisulfite, sodium or potassium sulfite or sodium or potassium thiosulfite. The copolymer reaction solution containing the predetermined amount of the reducing additive then is hydrolyzed to the corresponding acid form of the copolymer to provide the desired colorless, stabilized aqueous solution of maleic acid-C₁-C₅ alkyl vinyl ether copolymer which includes the reducing additive. The resultant copolymer solution contained about 11-13% solids therein.

While the additive could be added after hydrolysis, it is much preferred to add it directly to the anhydride copolymer before hydrolysis. The relative viscosity of the copolymer solution of Gantrez® AN-169 (methyl vinyl ether/maleic anhydride copolymer) from International Specialty Products Inc. thus obtained is much improved as compared to either the untreated copolymer or the treated copolymer after hydrolysis. The specific viscosity of the resultant copolymer solutions is about 8-13, as determined in water at a concentration of 1% at 25°C.

### COMPARATIVE EXAMPLES

### 1. Untreated Copolymer

A 1-liter resin kettle equipped with an agitator, a reflux condenser and a water bath was charged with 22.0 g. of methyl vinyl ether/maleic anhydride copolymer (Gantrez® AN-169, International Specialty Products Inc.) of specific viscosity of 2.6 (as determined in methyl ethyl ketone at a concentration of 1% at 25°C.) and 178.0 g. of deionized water. While agitating, the mixture was heated to 85°C. and maintained at this temperature for 1.5 hours. During this period the anhydride groups of the copolymer were converted to carboxylic acid groups generating a methyl vinyl ether/maleic acid copolymer. The clear solution which contained 12.4% solids was cooled to room temperature and maintained, in the dark, for 2 months. The stability of the solution was determined by measuring its specific viscosity (1% in H₂O, at 25°C.) and Brookfield viscosity (Spindle # 3, 5 rpm, 25°C.). The results were the following:

**TABLE 1**

| Time, months | Spec. Viscosity | Brookfield Viscosity, cps |
|---|---|---|
| 0 | 8.21 | 1250 |
| 2 | 2.02 | 360 |

The data shows that a solution of methyl vinyl ether/maleic acid copolymer, which did not contain any stabilizer, deteriorated to about 24% of its initial specific viscosity, and to about 29% of its initial Brookfield viscosity, after a period of 2 months.

### INVENTION EXAMPLES

### 1. Additive After Hydrolysis

To the solution of Comparative Example 1 various stabilizers were added. The stabilizing effect is apparent in the data in Table 2 below.

**TABLE 2**

| Additive/ ppm | Time, months | Spec. Viscosity | Brookfield Viscosity, cps |
|---|---|---|---|
| None | 0 | 8.21 | 1250 |
| | 2 | 2.02 | 360 |
| | | | |
| NaHSO₃/100 | 0 | 8.21 | 1250 |
| | 2 | 7.14 | 1120 |

The data shows an improvement over the untreated copolymer of Comparative Example 1. Specifically, the specific viscosity of the copolymer decreased by only 13% and the Brookfield viscosity of the copolymer solution decreased by only about 10% after standing for 2 months at room temperature.

### 2. Additive Before Hydrolysis

A copolymer solution as in Example 1 was prepared by dissolving Gantrez® AN-169 in an inorganic reducing additive and water prior to beginning of heating. The acid copolymer was made by heating as in Example 1. The results are shown in Table 3 below.

**TABLE 3**

| Additive/ppm | Time, months | Spec. Viscosity | Brookfield Viscosity, cps |
|---|---|---|---|
| NaHSO₃/50 | 0 | 10.16 | 2380 |
| | 2 | 9.68 | 2050 |
| | | | |
| NaHSO₃/100 | 0 | 12.28 | 2920 |
| | 2 | 10.37 | 2480 |

The data above demonstrates the advantage of adding the reducing additive during dissolution of copolymer. Accordingly, both the specific viscosity and the Brookfield viscosity values of the solution were at least 85% of its initial values after 2 months, and with much higher initial and final viscosities than when the same additive was used after hydrolysis.

## Claims

1. A colorless, stabilized aqueous solution of a copolymer of a C₁-C₅ alkyl vinyl ether and maleic acid suitable for use in denture adhesives which includes 10-500 ppm of an inorganic reducing additive which is sulfur dioxide or sodium or potassium sulfite, bisulfite, metabisulfite or thiosulfite, said stabilized solution exhibiting a retention of at least 85% of its original viscosity (Brookfield) after a period of 2 months.

2. A solution according to claim 1 wherein said solution is prepared by hydrolyzing a copolymer of a C₁-C₅ alkyl vinyl ether and maleic anhydride in the presence of said additive.

3. A solution according to claim 2 which includes 50-100 ppm of said additive.

4. A solution according to any one of the preceding claims containing 11-13% by weight solids.

## Patentansprüche

1. Farblose, stabilisierte, wäßrige Lösung eines Copolymers eines C₁-C₅-Alkylvinylethers mit Maleinsäure, die sich zur Verwendung in Haftmitteln für künstliche Gebisse eignet und 10-500 ppm eines anorganischen Reduktionsadditivs umfaßt, das Schwefeldioxid oder Natrium- oder Kaliumsulfit, -bisulfit, -metabisulfit oder -thiosulfit ist, wobei die stabilisierte Lösung nach 2 Monaten zumindest 85% ihrer ursprünglichen Viskosität (nach Brookfield) beibehält.

2. Lösung nach Anspruch 1, worin die Lösung durch Hydrolysieren eines Copolymers eines C₁-C₅-Alkylvinylethers mit Maleinsäureanhydrid in Gegenwart dieses Additivs hergestellt ist.

3. Lösung nach Anspruch 2, die 50-100 ppm dieses Additivs umfaßt.

4. Lösung nach einem der vorangegangenen Ansprüche, die 11-13 Gew.-% Feststoffe enthält.

## Revendications

1. Solution aqueuse stabilisée incolore d'un copolymère d'éther de vinyle et d'alkyle en C₁ à C₅/acide maléique, convenant pour être utilisée dans des adhésifs dentaires, qui contient de 10 à 500 ppm d'un additif réducteur minéral qui est le dioxyde de soufre ou le sulfite, le bisulfite, le métabisulfite ou le thiosulfite de sodium ou de potassium, ladite solution stabilisée conservant au moins 85% de sa viscosité (Brookfield) initiale après une durée de 2 mois.

2. Solution selon la revendication 1, dans laquelle ladite solution est préparée en hydrolysant un copolymère d'éther de vinyle et d'alkyle en C₁ à C₅/anhydride maléique en présence dudit additif.

3. Solution selon la revendication 2, qui contient de 50 à 100 ppm dudit additif.

4. Solution selon l'une quelconque des revendications précédentes, contenant de 11 à 13% en poids de solides.
